# EUROPEAN PATENT APPLICATION

(11) **EP 3 202 349 A1**
(43) Date of publication of application: **09.08.2017**
(21) Application number: 15846739.9
(22) Date of filing: 08.09.2015
(51) Int. Cl.: A61B 18/00

(54) **TREATMENT APPARATUS**

(30) Priority: 30.09.2014 JP 2014201279
(71) Applicant: Olympus Corporation, Hachioji-shi, Tokyo 192-8507 (JP)
(72) Inventor: ONAGA, Takeshi, Hachioji-shi, Tokyo 192-8507 (JP); FUNAKOSHI, Yasuo, Hachioji-shi, Tokyo 192-8507 (JP)
(74) Representative: Gunzelmann, Rainer
(86) International application number: PCT/JP2015/075459
(87) International publication number: WO 2016/052096

(57) **Abstract**

A treatment instrument (1) includes a grip (110) and a handle (160). The grip (110) is provided in an operation unit (100). The grip (110) includes a first surface (111) and a second surface (112) provided back to back. The handle (160) provided in the operation unit (100) on a distal end side of the grip (110). The handle (160) moves to the distal end side and the proximal end side relative to the grip (110). At least one of the first surface (111) and the second surface (112) is provided with a protrusion (121, 122).

## Description

### Technical Field

The present invention relates to a treatment instrument.

### Background Art

In general, a treatment instrument that holds body tissue, which is a treatment object, by an openable jaw and treats the body tissue is known. A mechanism in such a treatment instrument for opening and closing the jaw by a user holding a grip of an operation unit with a palm and a thumb, and operating a handle of the operation unit forward and backward with a middle finger, a ring finger, and a little finger, is known. For example, Jpn. Pat. Appln. KOKAI Publication No. 2010-540186 discloses a technology relating to an ultrasonic surgical treatment tool having such a mechanism. This ultrasonic surgical treatment tool includes a handle assembly which functions as the operation unit, holds body tissue by an end effector assembly which functions as a jaw, and treats the body tissue by ultrasonic vibration. To enable a user to perform precise treatment by using the above-described instrument, good operability of the operation unit is desired.

### Summary of Invention

An object of the present invention is to provide a treatment instrument including an operation unit with good operability.

According to an aspect of the present invention, a treatment instrument in which an operation of an end effector provided on a distal end side is operated by an operation unit provided on a proximal end side includes a grip provided in the operation unit and including a first surface and a second surface provided back to back; and a handle provided in the operation unit on a distal end side of the grip and configured to move on a reference surface to the distal end side and the proximal end side relative to the grip, the reference surface being an imaginary plane between the first surface and the second surface, wherein at least one of the first surface and the second surface is provided with a protrusion.

According to an aspect of the present invention, a treatment instrument includes a grip configured to be held between a palm and a thumb of a user when held by the user; and a handle including an opening in which at least one of an index finger, a middle finger, a ring finger and a little finger of the user is inserted when the handle is held by the user, and configured to move relative to the grip by bending and stretching of the finger inserted in the opening, wherein the grip is provided with a protrusion having a surface which is brought into contact with the thumb from a tip and a first joint of the thumb and which receives a force with which the thumb pushes the grip to the palm.

The present invention can provide a treatment instrument including an operation unit with good operability.

### Brief Description of Drawings

FIG. 1 is a schematic external view showing an exemplary configuration of a treatment instrument according to one embodiment;
FIG. 2A is a front view showing an example of the configuration of an operation unit;
FIG. 2B is a side view showing an example of the configuration of the operation unit;
FIG. 2C is a perspective view showing an example of the configuration of the operation unit;
FIG. 3 shows an example of the state where the operation unit is held;
FIG. 4A illustrates a position of a protrusion;
FIG. 4B illustrates a height of the protrusion;
FIG. 5A illustrates how force is transmitted when a handle is moved to the proximal end side;
FIG. 5B illustrates how force is transmitted when the handle is moved to the distal end side;
FIG. 6A is a front view showing another example of the configuration of the operation unit;
FIG. 6B is a side view showing another example of the configuration of the operation unit; and
FIG. 6C is a perspective view showing another example of the configuration of the operation unit.

### Description of Embodiments

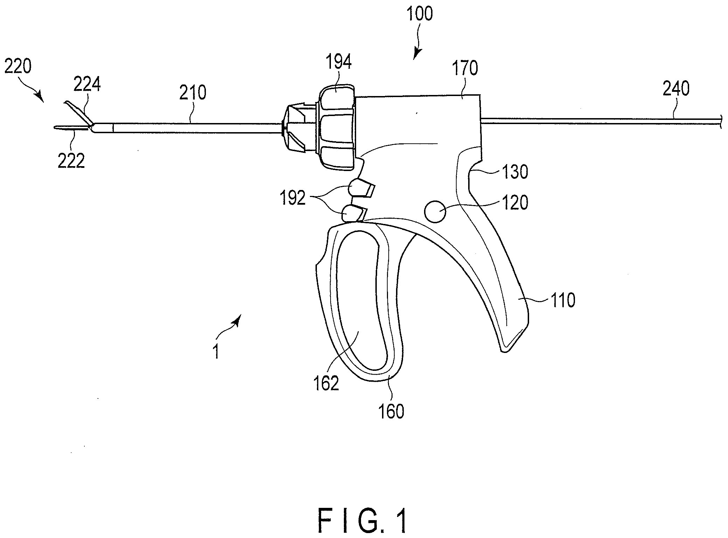

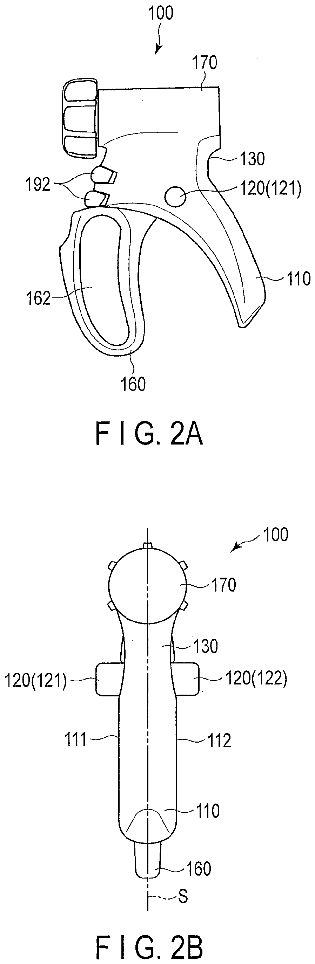

One embodiment of the present invention will be described with reference to the drawings. FIG. 1 is a schematic external view of a treatment instrument 1 according to the present embodiment. The treatment instrument 1 includes an operation unit 100, a columnar shaft 210 extending from the operation unit 100, and an end effector 220 provided on the distal end of the shaft 210. Hereinafter, the end effector 220 side is referred to as "the distal end side," and the operation unit 100 side is referred to as "the proximal end side" for explanations. The imaginary plane expanding on the plane of paper in FIG. 1 is defined as a reference surface S (see FIG. 2B). Accordingly, the central axis of the shaft 210 is on the reference surface S, and the shaft 210 is provided with the end effector 220 on the distal end side and the operation unit 100 on the proximal end side. The treatment instrument 1 is an instrument for holding body tissue, which is a treatment object, with the end effector 220 and performing treatment etc. on the body tissue, by a user operating the operation unit 100.

The operation unit 100 includes a grip 110 forming part of the main body of the operation unit 100. The operation unit 100 also includes a handle 160 that moves relative to the grip 110. The handle 160 is provided on the distal end side of the grip 110, and changes its position along the reference surface S toward the distal end side and toward the proximal end side with respect to the grip 110. The shape of the cross-section of the handle 160 on the reference surface S is annular. The inside of the ring of the handle 160 will be referred to as an opening 162. The shape of the handle 160 on the reference surface S includes a shape having an opening interposed between the distal end side surface and the proximal end side surface. This shape allows the force toward the distal end side and that toward the proximal end side to be easily exerted on the handle 160 by insertion of fingers in the opening 162, as will be described later.

The operation unit 100 includes a drive assembly 170 forming part of the main body of the operation unit 100. The positional relationship between the drive assembly 170 and the grip 110 is fixed. Namely, the exterior of the drive assembly 170 may be formed integrally with the grip 110, for example. The grip 110 may also be formed separately from the drive assembly 170 and fixed to the drive assembly 170. Hereinafter, the drive assembly 170 side of the operation unit 100 will be referred to as "the upper side," and the grip 110 end side will be referred to as "the lower side."

A rotation knob 194 is provided on the distal end side of the drive assembly 170. The shaft 210 is connected to the distal end side of the rotation knob 194. The rotation knob 194 is configured to rotate about the central axis of the shaft 210. As the rotation knob 194 rotates, the shaft 210 rotates about its central axis. As a result, the orientation of the end effector 220 changes.

A user wraps the grip 110 with the user's palm (hand palm) and thumb (pollex), and holds the operation unit 100 with one hand by inserting the user's middle finger (second finger), ring finger (third finger) and little finger (forth finger) in the opening 162 of the annular handle 160. At this time, sides of the thumb and index finger are placed on the upper side, i.e., the drive assembly 170 side, and a side of the little finger is placed on the lower side, i.e., the handle 160 end side (lower end side). A user changes the position of the handle 160 with respect to the grip 110 by moving the middle finger, the ring finger, and the little finger toward and away from the palm, i.e., by bending and stretching those fingers.

The operation unit 100 is provided with an output switch 192. The output switch 192 is arranged on the distal end side of the main body of the operation unit 100 between the handle 160 and the drive assembly 170. Namely, when a user holds the operation unit 100 as described above, the output switch 192 is arranged at a position where the user's index finger (forefinger; first finger) is placed.

The end effector 220 is provided with a first holding member 222 and a second holding member 224. The end effector 220 is configured to allow the first holding member 222 and the second holding member 224 to move relative to each other so as to open and close. Namely, the end effector 220 is configured to change the distance between the surface of the first holding member 22 facing the second holding member 224 and the surface of the second holding member 224 facing the first holding member 222. FIG. 1 shows a state where the first holding member 222 and the second holding member 224 are open. Accordingly, the end effector 220 can hold body tissue, which is a treatment object, by opening and closing of the first holding member 222 and the second holding member 224, i.e., by opening and closing of the end effector 220.

The opening and closing of the end effector 220 link to the relative movement between the grip 110 and the handle 160 by the mechanism in the drive assembly 170. Namely, when the handle 160 is moved away from the grip 110 as shown in FIG. 1, the end effector 220 opens. On the other hand, when the handle 160 is moved toward the grip 110, the end effector 220 closes. The movement of the handle 160 is transmitted to the first holding member 222 or the second holding member 224 of the end effector 220 by a mechanism using a publicly-known rod or wire passing through the shaft 210. Various methods including common ones may be adopted as the mechanism for linking the movement of the handle 160 including the drive assembly 170 to that of the end effector 220. Description of the mechanism is omitted herein.

The end effector 220 is provided with a mechanism for applying energy to held body tissue. For example, the treatment instrument 1 is configured to function as an ultrasonic treatment tool. In this case, the first holding member 222 is connected, via an ultrasonic wave transmitting member, to an ultrasonic transducer provided in the drive assembly 170. This configuration makes the first holding member 222 ultrasonically vibrate, and enables the treatment instrument 1 to cut body tissue held by the end effector 220 with the vibration.

The treatment instrument 1 may be configured to function as, for example, a high-frequency treatment tool. In this case, the first holding member 222 and the second holding member 224 are provided with a portion that functions as an electrode so that a high-frequency voltage is applied between the first holding member 222 and the second holding member 224. This configuration enables the first holding member 222 and the second holding member 224 to function as a bipolar electrode, and enables the treatment instrument 1 to cauterize body tissue held by the end effector 220.

The treatment instrument 1 may be configured to function as, for example, a heat treatment tool. In this case, the first holding member 222 and the second holding member 224 are provided with a heater. This configuration enables the treatment instrument 1 to cauterize body tissue held by the end effector 220.

The treatment instrument 1 may be configured to function as, for example, a stapler. In this case, the end effector 220 is provided with the function of a stapler, and the treatment instrument 1 is configured to conjugate body tissue held by the end effector 220.

The function provided to the tip of the end effector 220 may be a function other than the above, and may be a combination of various functions as described above. In addition, those functions may be unprovided. Namely, the treatment instrument 1 may be a mere forceps.

In the drive assembly 170, various elements are provided in accordance with the function provided to the end effector 220. For example, when the treatment instrument 1 functions as an ultrasonic treatment tool, an ultrasonic transducer is provided in the drive assembly 170. When the treatment instrument 1 functions as a high-frequency treatment tool, a drive circuit of a high-frequency voltage is provided in the drive assembly 170. When the treatment instrument 1 functions as a heat treatment tool, a drive circuit of a heater is provided in the drive assembly 170. The outer shape of the drive assembly 170 may be changed in accordance with the member provided in the drive assembly 170 as appropriate.

One end of a cable 240 is connected to the proximal end side of the drive assembly 170. The other end of the cable 240 is connected to a controller not shown. Energy is supplied from the controller to the ultrasonic transducer or the drive circuit in the drive assembly 170 via the cable 240. The aforementioned output switch 192 functions as a switch for switching between on and off of the energy output from the end effector 220.

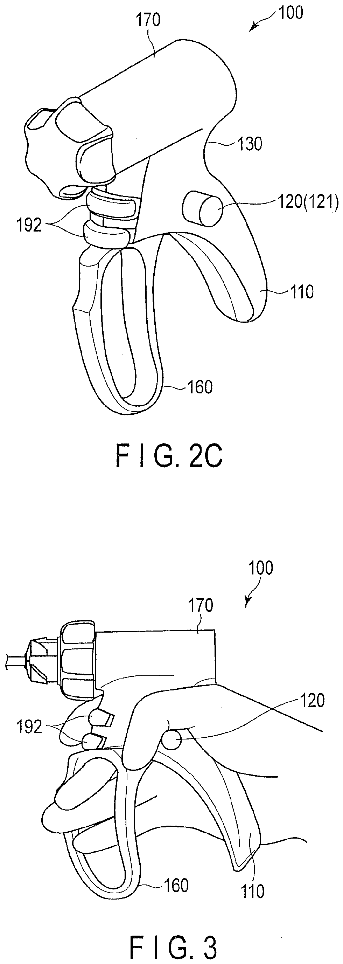

The configuration of the operation unit 100 will be further described. The configuration of the operation unit 100 is shown in FIGS. 2A, 2B, and 2C. FIG. 2A is a front view of the operation unit 100 viewed in a direction perpendicular to the reference surface S like in FIG. 1. FIG. 2B is a side view of the operation unit 100 viewed from the proximal end side on the reference surface S. FIG. 2C is a perspective view of the operation unit 100.

As shown in FIGS. 2A, 2B, and 2C, the main body of the operation unit 100 is provided with a protrusion 120 extending from its surface in a direction perpendicular to the reference surface S. The protrusion 120 is preferably formed between the handle 160 and a depressed portion 130 to be described later. Therefore, the protrusion 120 is preferably formed on the grip 110 constituting part of the main body of the operation unit 100. The protrusion 120 is provided on each side of the grip 110. Namely, as shown in FIG. 2B, a first protrusion 121, which is one of the protrusions 120, is provided on the left side, and a second protrusion 122, which is the other one of the protrusions 120, is provided on the right side, when viewed from the proximal end side to the distal end side. The protrusion 120 does not necessarily extend perpendicularly to the reference surface S, and may extend on the angle with respect to the reference surface S.

When the treatment instrument 1 is configured as a right-hand tool, i.e., when users operate the operation unit 100 only by their right hand, it is possible to provide only the first protrusion 121 without the second protrusion 122. Similarly, when the treatment instrument 1 is configured as a left-hand tool, i.e., when users operate the operation unit 100 only by their left hand, it is possible to provide only the second protrusion 122 without the first protrusion 121.

Accordingly, the grip 110 includes a first surface 111 and a second surface 112, which interpose the reference surface S therebetween. The first surface 111 is provided with the first protrusion 121, and the second surface 112 is provided with the second protrusion 122. The reference surface S, which is an imaginary plane, exists between the first surface 111 and the second surface 112, which are provided back to back with each other.

As shown in FIG. 2A, a depressed portion 130 is provided in the main body of the operation unit 100 on the proximal end side between the grip 110 and the drive assembly 170. The depressed portion 130 is formed by providing the base part of the grip 110 on the distal end side relative to the proximal end of the drive assembly 170. When a user holds the operation unit 100, the bases of the thumb and the index finger touch the depressed portion 130. As a result, the bases of the user's thumb and index finger are settled between the handle 160 and the drive assembly 170. At this time, the user's palm is brought into contact with the grip 110, and the upper portions of the index finger and the thumb are brought into contact with the drive assembly 170. The shape of the depressed portion 130 makes it easier to secure the operation unit 100 in the hand of the user when the operation unit 100 is held by the user.

FIG. 3 shows a state where a user holds the operation unit 100. As shown in the figure, the thumb of the user comes into contact with a protrusion 120. In particular, by the user bending the first joint of the thumb, the thumb comes into contact with the distal end side surface of the protrusion 120, i.e., the side surface facing the handle 160, from the tip to the first joint, and with the upper side surface of the protrusion 120 from the first joint to the second joint.

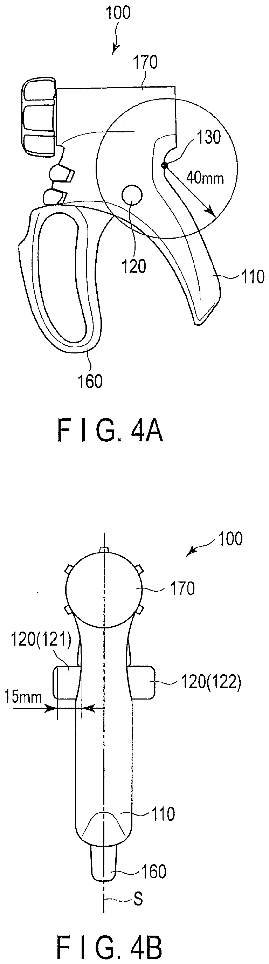

Next, an example of the dimension of the protrusion 120 will be described. As shown in FIG. 4A, the protrusion 120 is provided within, for example, 40 mm from an area of the depressed portion 130 where the web at the bases of the user's thumb and index finger touches. By providing the protrusion 120 at such a position, the thumb comes into contact with the distal end side surface of the protrusion 120 from the tip to the first joint, and with the upper side surface of the protrusion 120 from the first joint to the second joint.

FIG. 4B is a side view of the operation unit 100 viewed from the proximal end side. As shown in FIG. 4B, the height of the protrusion from the surface of the grip 110 is, for example, 15 mm. By providing the protrusion 120 at a height of approximately 15 mm, the thumb is firmly engaged with the protrusion 120. The above-described dimension of the protrusion 120 is an example, and is not limited to the described one.

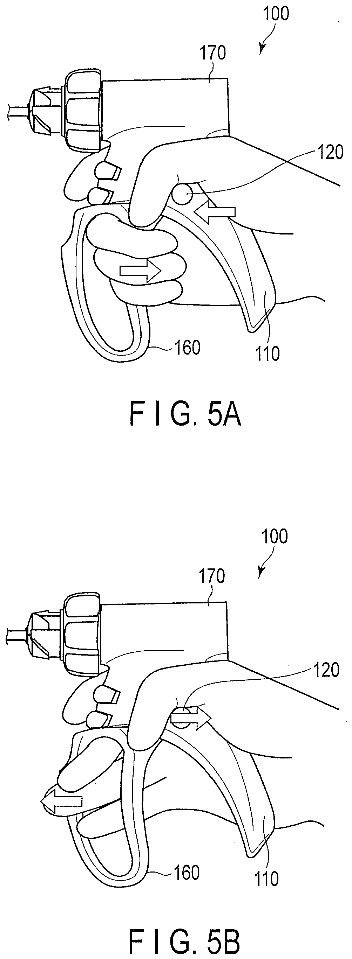

The advantage of providing the protrusion 120 on the surface of the grip 110 will be described. When the handle 160 is moved toward the grip 110, i.e., when the handle 160 is moved to the proximal end side, the operation unit 100 receives the following forces as shown in FIG. 5A. A grip 110 side portion of the handle 160 is pushed to the grip 110 side by the palm sides of the middle finger, ring finger, and little finger. At this time, the proximal end side surface of the grip 110 is pushed to the handle 160 side by the palm, in particular, the thenar eminence. Accordingly, the force with which a user closes their hand, i.e., the force with which a user bends their middle finger, ring finger, and little finger, is efficiently transmitted to move the handle 160 toward the grip 110.

When the handle 160 is moved away from the grip 110, i.e., when the handle 160 is moved to the distal end side, the operation unit 100 receives the following forces as shown in FIG. 5B. A distal end side portion of the handle 160 is pushed to the distal end side by the back sides of the middle finger, ring finger, and little finger. At this time, the distal end side surface of the protrusion 120 of the grip 110 is pushed to the proximal end side by the palm side of the thumb from the tip to the first joint. Namely, the presence of the surface of the protrusion 120 directed to the distal end side (directed to the handle 160) and receiving the force with which the thumb pushes the grip 110 to the proximal end side enables the thumb engaged with this surface of the protrusion 120 to efficiently exert the force with which the grip 110 is pushed to the proximal end side on the grip 110. Accordingly, the force with which a user opens their hand, i.e., the force with which a user stretches their middle finger, ring finger, and little finger is efficiently transmitted to move the handle 160 to the distal end side.

Let us assume the case where the protrusion 120 does not exist. The circumstances under which the handle 160 is moved to the distal end side in that case will be considered. At this time, the force with which a user's hand pushes the grip 110 to the proximal end side is not effectively transmitted to the grip 110. This is because the grip 110 does not have any surface directed to the distal end side and receiving the force with which a palm and a thumb pushes the grip 110 to the proximal end side. Therefore, when the protrusion 120 does not exist, the grip 110 is secured in the palm by the force with which the palm and the thumb hold the grip 110 therebetween, and this force is utilized to exert the pushing force to the distal end side on the grip 110. At this time, the hand easily slips on the grip 110, and the slip does not allow the hand opening force to be efficiently transmitted to the grip 110 or the handle 160.

Accordingly, the protrusion 120 of the present embodiment improves force transmitting efficiency in transmitting the hand force to the grip 110 and the handle 160 when moving the handle 160 to the distal end side.

In the above-described example, the protrusion 120 is located below the depressed portion 130. However, the position of the protrusion 120 is not limited to that position. The protrusion 120 may be arranged above the depressed portion 130, i.e., on the drive assembly 170 side with respect to the depressed portion 130.

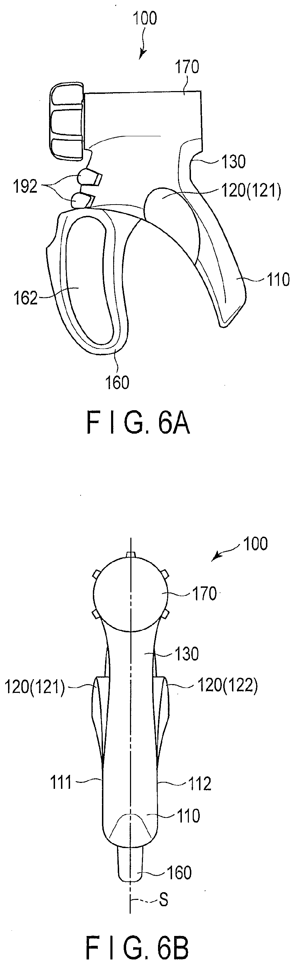

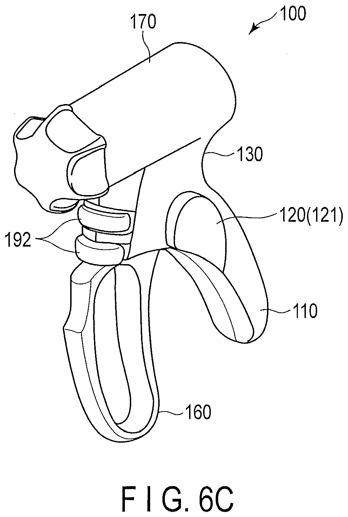

Described above is an example where the protrusion 120 is circular. However, the shape is not limited to this. For example, as shown in FIGS. 6A to 6C, the protrusion 120 may extend along the grip. In addition, the shape of the protrusion 120 may be another shape, such as an oval, a square, or a triangle. However, the protrusion 120 preferably has an upper side surface that the user's thumb touches from the first joint to the second joint, and a distal end side surface that the user's thumb touches from the tip to the first joint. Namely, the protrusion 120 preferably has a shape including a portion where the direction of the normal to the side surface of the protrusion 120, i.e., the component of the normal to the protrusion 120 projected on the reference surface S, is the upper direction on the proximal end side and changes to the distal end direction on the distal end side.

In particular, the protrusion 120 preferably has a distal end side surface that the user's thumb touches from the tip to the first joint. Namely, the protrusion preferably has a surface directed to the handle 160.

The protrusion 120 may have any size. Namely, as described above, as long as the protrusion 120 allows the user's thumb to be engaged therewith, the other portions of the protrusion 120 may have any shape or size, and in particular, the shape of the lower part is not limited.

In the above embodiment, described is an example where the end effector 220 opens and closes in accordance with an operation of the handle 160. However, the configuration of the treatment instrument 1 is not limited to this. For example, the treatment instrument 1 may be configured to allow the shaft to bend in the end effector in accordance with an operation of the handle 160. For example, when the treatment instrument 1 functions as a stapler, the treatment instrument 1 may be configured to punch out a staple in accordance with an operation of the handle 160. In addition, another operation may be performed in accordance with an operation of the handle 160. As described above, the end effector may include any mechanism on the distal end side of the shaft.

## Claims

1. A treatment instrument in which an operation of an end effector provided on a distal end side is operated by an operation unit provided on a proximal end side, the treatment instrument comprising:
a grip provided in the operation unit and including a first surface and a second surface provided back to back; and
a handle provided in the operation unit on a distal end side of the grip and configured to move on a reference surface to the distal end side and the proximal end side relative to the grip, the reference surface being an imaginary plane between the first surface and the second surface,
wherein at least one of the first surface and the second surface is provided with a protrusion.

2. The treatment instrument according to claim 1,
wherein
the operation unit further includes a drive assembly constituting part of a mechanism for transmitting the relative movement between the grip and the handle to the end effector, and a depressed portion between the grip and the drive assembly on a proximal end side of the operation unit, and
the protrusion is provided apart from the depressed portion.

3. The treatment instrument according to claim 2,
wherein the drive assembly, the depressed portion, and the protrusion are arranged in an order of appearance in a direction perpendicular to a line connecting the distal end side and the proximal end side.

4. The treatment instrument according to claim 1,
wherein
the operation unit further includes a drive assembly constituting part of a mechanism for transmitting the relative movement between the grip and the handle to the end effector,
the protrusion is provided apart from the drive assembly, and
the protrusion has a shape including a portion where a direction of a component of a normal to the protrusion projected on the reference surface is a direction of the drive assembly on the proximal end side and changes to a direction of the distal end side on the distal end side.

5. The treatment instrument according to claim 1,
wherein the protrusion has a surface directed to the distal end side.

6. The treatment instrument according to claim 1,
wherein a shape of the handle on the reference surface includes a shape having an opening interposed between the distal end side surface and the proximal end side surface.

7. A treatment instrument comprising:
a grip configured to be held between a palm and a thumb of a user when held by the user; and
a handle including an opening in which at least one of an index finger, a middle finger, a ring finger and a little finger of the user is inserted when the handle is held by the user, and configured to move relative to the grip by bending and stretching of the finger inserted in the opening,
wherein the grip is provided with a protrusion having a surface which is brought into contact with the thumb from a tip and a first joint of the thumb and which receives a force with which the thumb pushes the grip to the palm.
